# EUROPEAN PATENT APPLICATION

(11) **EP 0 521 686 A1**
(43) Date of publication of application: **07.01.1993**
(21) Application number: 92305997.6
(22) Date of filing: 29.06.1992
(51) Int. Cl.: C07D 263/52, C07C 271/22, C07C 269/06, C07C 237/20

(54) **Stereoselective production of hydroxyamide compounds from chiral a-amino epoxides**

(30) Priority: 02.07.1991 US 724617; 02.07.1991 US 724671
(71) Applicant: MERCK & CO. INC., Rahway New Jersey 07065-0900 (US)
(72) Inventor: Askin, David, Warren NJ 07059 (US); Volante, Ralph, East Windsor, NJ 08520 (US); Wallace, Michael, Matawan, NJ 07747 (US); Shinkai, Ichiro, Westfield, NJ 07090 (US)
(74) Representative: Thompson, John Dr.

(57) **Abstract**

An efficient process for stereoselective preparation of a medicinally significant hydroxyamide compound of structural formula: 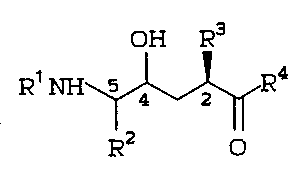
comprises the addition of metalated amide enolates to chiral α-amino metalated epoxides. The hydroxyamide reaction products are useful as inhibitors of the HIV protease or of renin, or as intermediates in the preparation of inhibitors of the HIV protease or renin.

## Description

### BACKGROUND OF THE INVENTION

The present invention concerns the diastereoselective synthesis of hydroxyethylene dipeptide isosteres **III** by a novel route which involves the addition of the amide **II** to the epoxide **I** by treatment with strong base at low temperatures, as shown below in Scheme I.

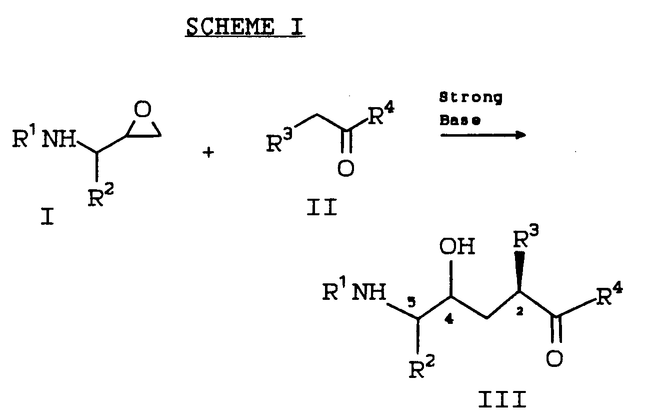

Hydroxyethylene dipeptide isosteres are medicinally important enzyme (renin and HIV protease) inhibitors. In particular, 2R, 4S, 5S diastereomeric hydroxyamide isosteres have been pursued and synthesized as intermediates in the preparation of such enzyme inhibitors, as well as being end-product inhibitors themselves. See, e.g., Vacca, J.P., et al., J. Med. Chem., 34:1228 (1991).

In the past, entry into the the synthesis of hydroxyethylene dipeptide isosteres involved alkylation of a lactone, derived from an aldehyde, DeCamp, A.E., et al., Tetrahedron Lett., 32: 1867 (1991), with various aryl halides to afford the trans-alkylated lactone products. Evans, B.E., et al., J. Org. Chem., 50: 4615 (1985); Fray, A.H., et al., J.Org. Chem., 51: 4828 (1986).

In the prior art, a four step coupling procedure is necessary to effect amide bond formation from the alkylated lactone for the synthesis of the isosteres of structure **III**, which is a major problem with this route. The alkylated lactone is saponified to afford an intermediate hydroxy-acid which is then protected at the hydroxyl group to afford the carboxylic acid intermediate. The carboxylic acid intermediate is then coupled with an amine to afford the hydroxy protected isostere which is finally deprotected to afford the isostere **III**. In addition to being lengthy, the process requires expensive reagents to activate the carboxylic acid, is operationally difficult on a large scale and results in some recovery of the alkylated intermediate lactone after the amide bond formation step. Thus, a new route to the hydroxyethylene dipeptide isosteres that does not involve the lengthy and ineffecient conversion of an alkylated lactone to the desired isostere **III** would be superior to existing methodology.

The work of Marshall, et al., J. Org. Chem. 50: 1602 (1985), has shown that epoxides bearing α-(metalloalkoxide) groups are much more reactive than the corresponding epoxides bearing α-ether groups. However, no examples exist of the reaction of epoxides bearing α-(metallo-amino) or α-[metallo-(N-t-butoxycarbonyl-amino)] groups with metalated amide enolates.

Experiments involving the reaction of the metalated amide enolate derived from compound **II** in Scheme I with simple unsubstituted epoxides show that a substantial rate acceleration is gained via epoxide activation by the α-metalated amino group.

Although metalated chiral amide enolates have been previously used to form 2-alkyl-4-hydroxycarboxamide products by reaction with chiral epoxides [Askin, D., et al., Tetrahedron Lett., 29: 4245 (1988)], metalated chiral amide enolates have not previously been used to make hydroxyethylene dipeptide isosteres **III** by reaction with α-N-metalated chiral epoxides.

The present invention has several advantages over the prior art for making medicinally important intermediates and end-product compounds useful for the treatment of serious diseases. Most importantly, the novel process of the invention provides a rapid entry into hydroxyethylene dipeptide isosteres by circumventing the problematic four-step coupling/amidation sequence. Thus, the invention is a more economical, operationally practical and efficient process for the construction of hydroxy-ethylene dipeptide isosteres than previous processes. This invention provides a direct, high yielding, stereoselective route to hydroxyamide compounds useful in the preparation of diastereomeric compounds having the ability to inhibit certain proteolytic enzymes, including renin and the protease of human immunodeficiency virus (HIV).

### SUMMARY Of THE INVENTION

The novel process of the invention is summarized according to SCHEME I: 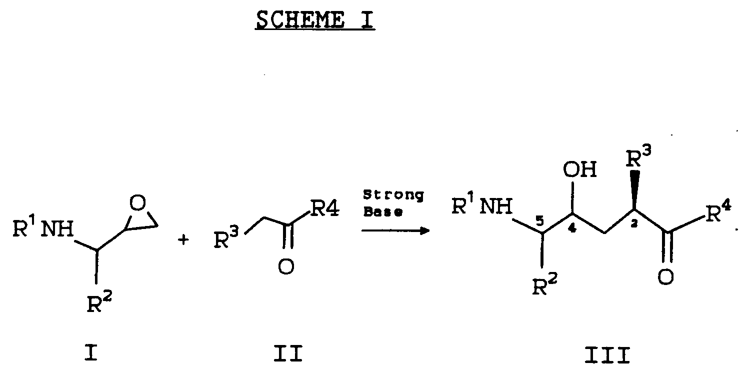
whereby an epoxide of structural formula **I** and an amide of structural formula **II** are reacted by treating **I** and **II** with strong base at low temperatures. The strong base effects metalation of the amino group of **I** and metalation of the amide **II** at the position a-to the carbonyl group to afford the reactive metal amide enolate which then effects ring opening of the metalated epoxide at the terminal position to afford the product **III**. A new center of asymmetry is created in the product **III** at the 2-position. The reaction may be run in one or two steps. Compounds comprehended by **III** are useful in the preparation of inhibitors of proteolytic enzymes, especially of the HIV protease, and of renin, and some of the compounds of formula **III** are themselves useful inhibitors of the HIV protease.

### DETAILED DESCRIPTION OF THE INVENTION

This invention provides a direct, high yielding, stereoselective route to hydroxyamide compounds via metallo-enolate methodology. The process is represented in Scheme I as the addition of the amide **II** to a chiral epoxide **I** by treatment with strong base at low temperature to yield the hydroxyamide **III**. Nearly exclusive formation of a single diastereomer of **III** results from this reaction scheme: 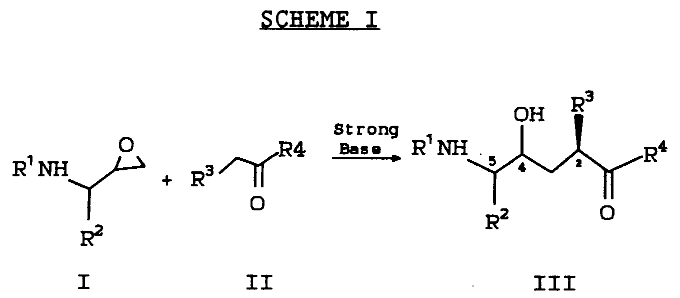
at low temperatures in an etherial solvent, wherein:
R¹ is:
a) t-Boc, Aoc, Adc, Mcb, Mch, Cbz, Alloc, or Fmoc,
b) benzyl,
c) lower alkyl,
d) CH₃O(CH₂CH₂O)ₙCO-, wherein n is from 1 to 10,
e) tetrahydrofuranyloxy-CO-, or
f) R-CO-, wherein R is an amino acid;

R² is:
a) -H,
b) benzyl,
c) cyclohexyl-CH₂-,
d) isobutyl,
e) benzyl-O-benzyl-,
f) cinnamyl-, or
g) parahydroxybenzyl;

R³ is:
a) -H,
b) lower alkyl,
c) phenyl,
d) benzyl,
e) -CH₂-phenyl-O-R⁵, or
f) -CH₂-CH=CH-phenyl-O-R⁵;

R⁴ is:
a) 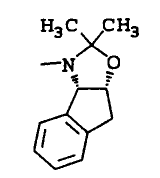
b) 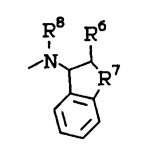 wherein:
   R⁶ is -H, or -OH;
   R⁷ is -CH₂-O-, or 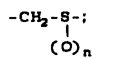 n = 0, 1, or 2;
c) 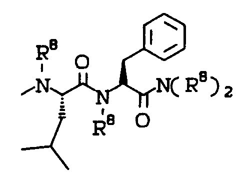
d) 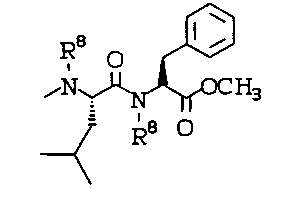
e) 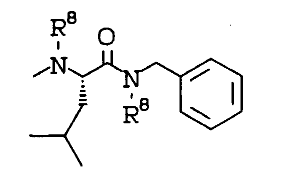
f) 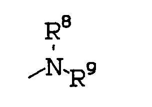
g) 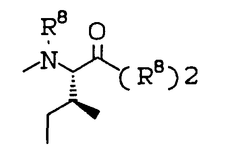 or
h)

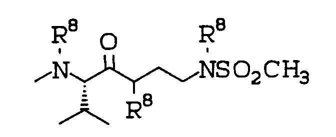

R⁵ is:
a) -H
b) benzyl,
c) -CH₂CH₂-OH, or
d)

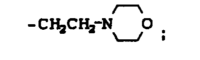

R⁸ is a removable protecting group selected from among:
a) t-Boc, Aoc, Adc, Mcb, Mch, Cbz, Alloc, or Fmoc, or
b) benzyl; and

R⁹ is:
a) lower alkyl, or
b) benzyl.

The strong base must be a metal-containing base. The strong base may or may not be in an inert anhydrous organic solvent, such as, e.g., cyclic or acyclic hydrocarbons including hexane, pentane, cyclohexane, etc. Suitable strong bases include n-butyllithium (n-BuLi), s-BuLi, t-BuLi, potassium tert-butoxide, lithium diisopropylamide (LDA), lithium isopropylcyclohexylamide, lithium pyrrolidide, lithium tetramethylpiperidide, phenyllithium, isopropylmagnesium chloride, isobutylmagnesium chloride, and other similar strong bases known in the art. Preferred strong bases are n-Buli, s-Buli, isopropyl magnesium chloride and LDA, with n-BuLi and isopropyl magnesium chloride being most preferred.

The strong base employed in Scheme I effects metalation of the amino group of epoxide **I** and metalation of the amide **II** at the position α to the carbonyl group to afford the reactive metal amide enolate which then effects ring opening of the metalated epoxide at the terminal position to afford the product **III**. A new center of asymmetry is created in the product isostere **III** at the 2-position. A surprising finding in this invention is that for certain chiral epoxides **I** and chiral amides **II**, nearly exclusive formation of a single diastereomeric isostere **III** results. Thus, a key aspect of the invention concerns the high selectivity in the formation of the 2-stereocenter of **III** (with the desired stereochemistry) in the condensation of the metalated amide enolate derived from **II** (where R⁴ may or may not possesses chirality) with electrophiles such as the metalated epoxide derived from **I** (i.e., where the -NH- of epoxide **I** = -N(metal)-).

The reaction of Scheme I is preferably run at a low temperature, for example ranging between about -82°C and 0°C. To effect metalation of the epoxide **I** and amide **II**, the temperature range is maintained more preferably between about -82°C and -65°C, and most preferably between about -80°C and -75°C, and to effect the reaction of the metalated derivatives of **I** and **II** to form **III**, the temperature range is maintained more preferably between about -50°C and -10°C, and most preferably between about -30°C to -20°C.

The etherial solvents are any solvents suitable for use in Scheme 1, including, e.g., tetrahydrofuran, 1,2-dimethoxyethane, di-ethyl ether and methyl-t-butyl ether, with tetrahydrofuran being preferred.

It is to be understood that the nitrogen protecting groups provided for in the definitions above may be removed to generate free amino compounds. Thus, the R⁸ group, wherever it appears, may be removed according to methods known in the art. Furthermore, the cyclic-aminal protecting acetonide (also called isopropylidene), present in the definition of R⁴ part (a), may similarly be removed.

As used herein, lower alkyl means straight or branched chain alkyls of one to five carbon atoms. When any one variable occurs more than one time in a molecule, its definition on each occurrence is independent of its definition at every other occurrence. Ph stands for phenyl, Bn for benzyl, and Et for ethyl. Aoc, t-Boc, etc., are amino or urethane protecting groups known in the art; t-Boc is preferred as it is easily removed under mild conditions. As used herein, the term amino acid encompasses all naturally ocurring amino acids.

The novel process of the present invention can be done in either one step or two steps. The two-step procedure involves the metalation of the epoxide **I** with a strong base at reduced temperatures, and the separate metalation of the amide **II** with the same or a different strong base at reduced temperatures, followed by addition of the metalated epoxide and the metalated amide enolate to produce the hydroxyamide product **III**. Alternatively, the epoxide and the amide can both be metalated and reacted in one step by addition of approximately 2 to 3 molar equivalents of a strong base to a solution containing about a 1:1 molar equivalent of both the epoxide **I** and the amide **II** to produce **III**.

In a preferred embodiment of the novel process, shown below in Scheme II, the chiral epoxide **IV** is metalated with a strong base, preferably isobutylmagnesium chloride, n-BuLi, s-BuLi, or LDA, and most preferably isobutylmagnesium chloride or n-BuLi, to produce the α-[metallo-(N-t-Boc-amino)]-epoxide **V**, by treating **IV** with the strong base at low temperatures.

Addition of **V** to the metalated amide enolate derived from treatment of **II** with strong base at low temperatures provides **VI** almost exclusively as the 2R, 4S, 5S diastereomer. The amide **II** is converted to the metalated amide enolate preferably by treatment with n-BuLi, s-BuLi or LDA, and most preferably with n-BuLi.

The 2R, 3S epoxide **IV** is prepared from commercially available amino acids or N-protected amino acids according to standard techniques well known in the art to one of ordinary skill such as, for example, the procedure disclosed in Luly, et al., J. Org. Chem, 52:1487 (1987).

The amide **II** is prepared, for example, from condensation of commercially available 3-phenylpropionic acid (also known as hydrocinnamic acid), substituted or unsubstituted on the phenyl ring, with an amine of formula H-R⁴, where R⁴ is as previously defined, and R⁴ may or may not have protecting groups on it. H-R⁴ is preferably (1S,2R)cis-1-amino-2-indanol, which can be protected as the acetonide once the amide coupling is complete (see Example 2). (1S, 2R)-Cis-1-amino-2-indanol is prepared from commercially available indene according to standard techniques well known in the art to one of ordinary skill such as, for example, the procedures disclosed in Hassner, et al., J. Org. Chem., 32:540 (1966), and resolved according to standard techniques well known in the art. 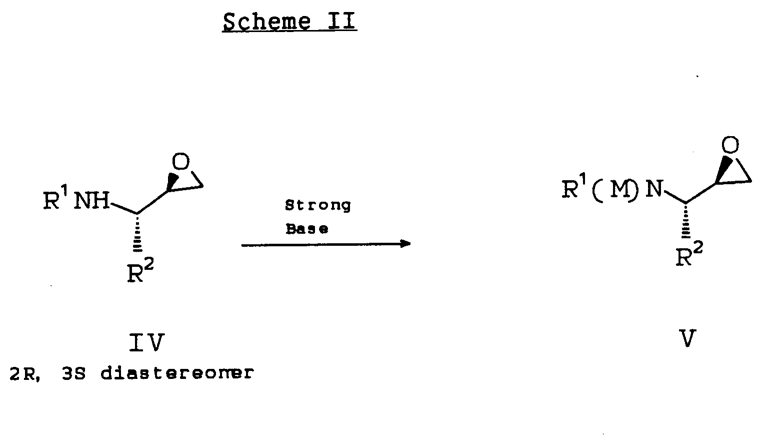 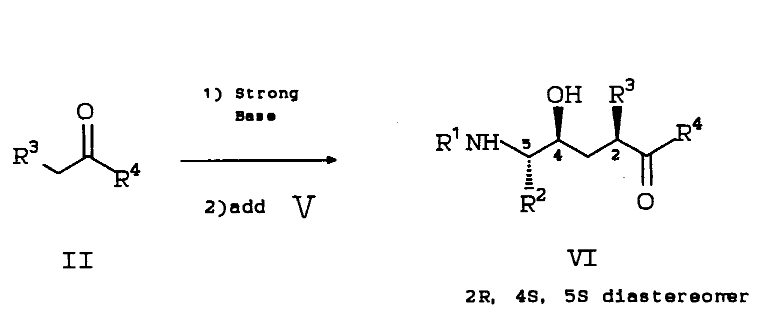
wherein M represents the metal or metal group that corresponds to the metal or metal group contained in the strong base chosen for use with the epoxide **IV** in the procedure. For example, when n-BuLi is the strong base used to treat **IV**, M is Li; when isopropyl magnesium chloride is used, M is MgCl.

In another preferred embodiment of the novel process, shown below in Scheme III, approximately 1:1 molar equivalents of epoxide **IV** and amide **II** are combined in one pot with appropriate solvent and reacted by treatment with approximately 2 to 3 molar equivalents of strong base, preferably n-BuLi, s-BuLi, or LDA and most preferably n-BuLi, to form the corresponding α-metallo (N-t-Boc-amino) epoxide and metallated amide enolate. The reaction yields **VI** almost exclusively as the 2R, 4S, 5S diastereomer.

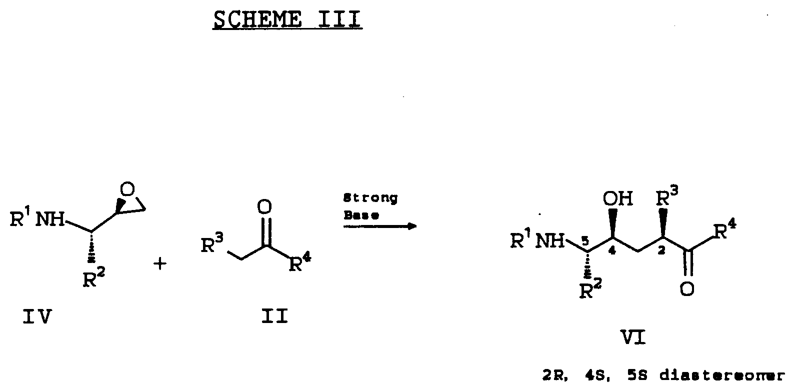

The compounds produced by the novel process of this invention are useful as intermediates in the preparation of peptides having defined stereochemistry. Furthermore, compounds having in vitro or in vivo renin or HIV protease inhibitory activity may be prepared directly according to the disclosed process.

Thus, in another preferred embodiment of this invention, the following 2R, 4S, 5S diastereomeric hydroxyamide compounds of structual formula **X** described in Table I, useful as inhibitors of the HIV protease, are prepared from the corresponding epoxide **VII** and amide **VIII** starting materials, also described in Table I. Each compound of Table I is prepared from reaction of the corresponding α-[metallo-(N-t-Boc-amino)]-epoxide with the corresponding metalated amide enolate according to either Scheme II or Scheme III, followed by deprotection of the novel intermediate **IX** to the amide and hydroxyl containing compound **X** (see Example 8).

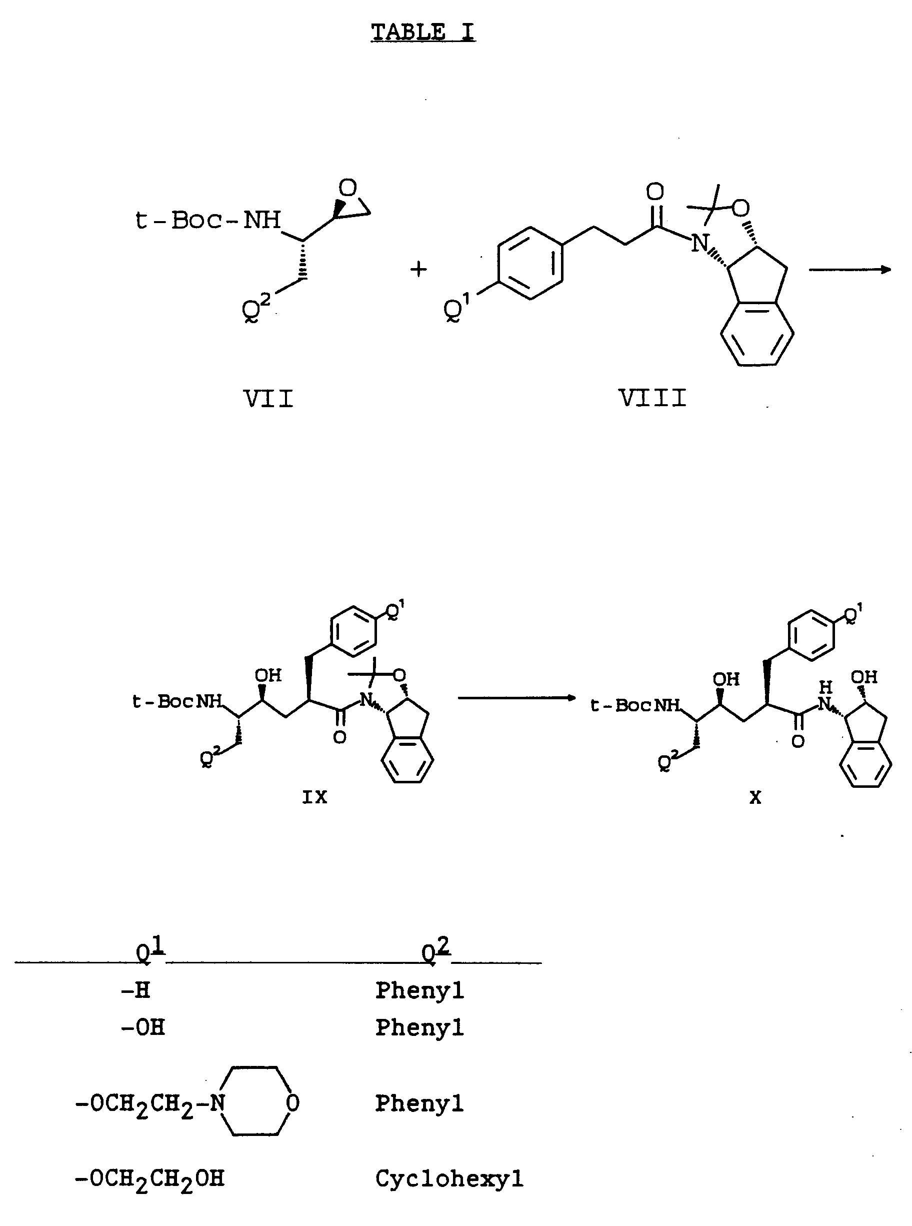

The novel process of this invention is useful to prepare diastereomeric compounds for in vitro testing as inhibitors of the HIV protease, renin, or other proteases of interest.

Furthermore, HIV protease or renin inhibitory compounds produced by this process may be administered to patients in need of such treatment in pharmaceutical compositions comprising a pharmaceutical carrier and therapeutically-effective amounts of the compound or a pharmaceutically acceptable salt thereof.

Representative experimental procedures utilizing the novel process are detailed below. These procedures are exemplary only and should not be construed as being limitations on the novel process of this invention.

### EXAMPLE 1

### Preparation of Amide 4

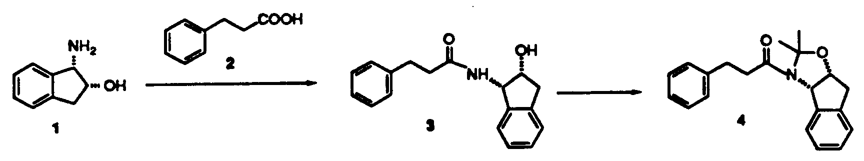

Hydrocinnamic acid (**2**, 50.4 g, 0.336 mol) was dissolved in 400 mL of dry THF and cooled to 0°C. Triethylamine (49.0 mL, 0.352 mol) was added followed by pivaloyl chloride (41.3 mL, 0.335 mol) which was added over a 25 min period. The mixture was aged at 0°C for 1h. The amino-alcohol **1** (50.0 g, 0.335 mol) (prepared according to Hassner, A. et al., J. Org. Chem. 1966, 32: 540) was then dissolved in 750 mL THF and added to the 0°C reaction mixture over a 1 h period. The mixture was then warmed to 25°C and aged for 2.5 h. The reaction was partitioned with 1000 mL ethyl acetate and 500 mL water. The layers were separated and the aqueous layer extracted once with 500 mL of ethyl acetate. The combined organic layers were washed with 400 mL water, 400 mL aqueous sodium bicarbonate, 400 mL saturated sodium sulfate and dried (MgSO₄). The dried organic phase was concentrated in vacuo to afford the crude amide **3** which was slurried in 900 mL of CH₂Cl₂ at 25°C. To this suspension was added 2-methoxypropene (38.8 mL, 0.405 mol) followed by pyridinium paratoluenesulfonate (8.4 g, 34 mmol). The resulting pale yellow solution was aged at 25°C for 5.5 h, then poured into 600 mL of saturated aqueous sodium bicarbonate. The layers were separated and the aqueous layer was extracted with 250 mL of CH₂Cl₂. The combined organic phase was washed with 100 mL saturated aqueous sodium bicarbonate, 100 mL water, and dried over MgSO₄. The volatiles were removed in vacuo to afford 82 g of crude product that was dissolved in 50 mL of THF at 50°C and cooled to 25°C. Hexane (275 mL) was then added over 45 min to the stirred slurry which was then cooled to 0°C and filtered. The product was washed with 50 mL of cold hexane and dried at high vacuum to afford 79.25 g (74% overall) of the acetonide amide derivative 4 as a white solid.
¹³C NMR (75.5 MHz, CDCl₃, major rotamer) δ_{c} 168.8, 140.9, 140.8, 140.6, 128.6, 128.5, 128.4, 127.1, 126.3, 125.8, 124.1, 96.5, 78.6, 65.9, 38.4, 36.2, 31.9, 26.5, 24.1.

### EXAMPLE 2

### Preparation of phenol-amide 8

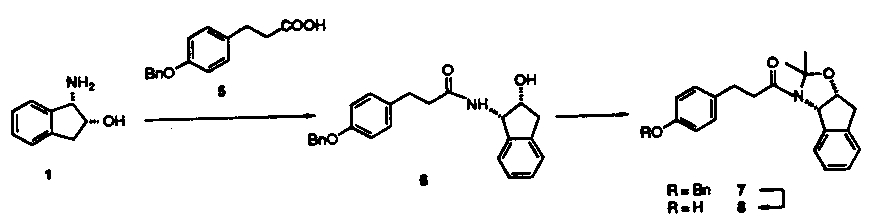

To a solution of 3-(4-Benzyloxy-phenyl)-propionic acid (**5**, 10.1 g, 39.4 mmol) in 260 mL sieve dried DMF at 25°C was added the amino-alcohol **1** (5.88g, 39.4 mmol), HOBT-hydrate (6.39g, 47.3 mmol), EDC (11.3g, 59.1 mmol) and 1-methyl-piperidine (7.18 mL, 59.1 mmol). The resulting mixture was aged at 25°C for 21 h, then the volatiles were removed by concentration in vacuo. The crude residue was diluted with 250 mL of ethyl acetate and washed with 100 mL of water. The aqueous layer was back-extracted with ethyl acetate (2 x 100 mL), and the combined organic layer was washed with 100 mL 10% citric acid, 100 mL of water, 100 mL of saturated aqueous NaHCO₃, 100 mL of saturated aqueous NaCl. The organic phase was dried over MgSO₄ and concentrated in vacuo to afford 14.6 g of crude amide **6**. A portion of the crude amide **6** (6.94 g, 17.9 mmol) was subdivided and dissolved in 50 mL of CH₂Cl₂ at 25°C and 2-methoxypropene (3.43 mL, 35.8 mmol) was added followed by pyridinium paratoluenesulfonate (517 mg, 2.06 mmol) and the resulting solution was aged at 25°C for 19 h. The reaction was quenched with 110 mL of saturated aqueous NaHCO₃ and the layers were separated, and the aqueous phase was back-extracted with CH₂Cl₂ (2 x 100 mL). The combined organic phase was washed with water (50 mL), dried with MgSO₄ and concentrated in vacuo to afford 8.15 g of crude product that was dissolved in 50 mL of ethyl acetate and heated to reflux, then cooled to 25°C and diluted with 100 mL of hexane while stirring over 1 h, then cooled to -20°C and aged overnight. The resulting slurry was filtered and washed with cold (3:1) hexane/ethyl acetate and dried at high vacuum to afford 4.93 g of the acetonide amide derivative **7** as an off white solid. The compound **7** (2.76 g, 6.46 mmol) and triethylamine (940 µL, 6.7 mmol) were dissolved in 20 mL THF and 450 mg of 10% palladium on carbon was added. The resulting suspension was shaken on a paar shaker apparatus under 40 psi of hydrogen for 2.5 h, then filtered , thru celite and concentrated in vacuo to afford 2.18 g of the phenol **8** as a foam.
¹³C NMR (75.5 MHz, CDCl₃, major rotamer) δ_{c} 170.0, 155.2, 140.6, 140.4, 131.7, 129.6, 128.6, 127.2, 125.8, 124.1, 115.5, 96.7, 78.7, 66.0, 38.8, 36.2, 31.4, 26.5, 24.0.

### EXAMPLE 3

### Preparation of morpholinoethyl amide 9

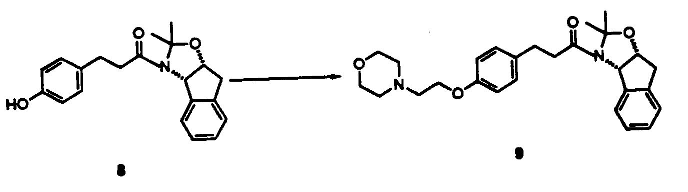

To a solution of crude phenol-amide **8** (2.18 g, 6.46 mmol) in 82 mL sieve dried CH₃CN at 25°C was added powdered K₂CO₃ (2.23 g, 16.1 mmol) and N-(chloroethyl)morpholine hydrochloride (1.26 g, 6.77 mmol). The resulting slurry was heated to 80°C and aged for 17 h. The mixture was then cooled to 25°C and partitioned with 100 mL water and 200 mL ethyl acetate and the layers were separated. The aqueous layer was extracted with ethyl acetate (2 x 500 mL) and the combined organic layer was washed with saturated aqueous NaCl and dried over MgSO₄. The organic phase was concentrated in vacuo to afford 3.19 g of a crude product that was purified by chromatography on 160 g of silica gel. Elution with 5% methanol in ethyl acetate gave 2.80 g (94% from benzyl ether **7**) of the desired morpholinoethyl amide **9** as a gum.
¹³C NMR (75.5 MHz, CDCl₃, major rotamer) δ_{c} 168.9, 157.2, 140.8, 140.6, 133.2, 129.5, 128.4, 127.1, 125.8, 124.1, 114.6, 96.5, 78.6, 66.8, 65.9, 65.7, 57.6, 54.0, 38.6, 36.2, 31.0, 26.5, 24.1.

### EXAMPLE 4

### Preparation of epoxide 11

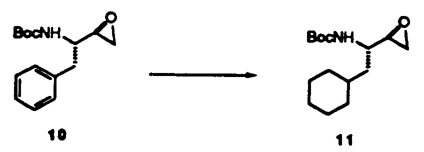

To a solution of epoxide **10** (1.00 g, 3.81 mmol) (prepared according to Luly, J.R., et al., J. Org. Chem. 1987, 52: 1487) in 30 mL of ethyl alcohol at 25°C was added 200 mg of 5% rhodium on alumina. The resulting mixture was hydrogenated in a shaker bomb at 25°C at 500 psi for 22 h. The mixture was then filtered thru celite and concentrated in vacuo to afford a crude product that was purified by chromatography on 55 g of silica gel. Elution with hexanes/ethyl acetate (7:1) gave 981 mg (95%) of the cyclohexyl-epoxide **11** as a colorless oil.
¹³C NMR (75.5 MHz, CDCl₃) δ_{c} 155.7, 79.2, 54.0, 46.5, 44.4, 40.8, 34.1, 33.8, 32.8, 28.3, 26.5, 26.3, 26.1.

### EXAMPLE 5

### Preparation of isostere acetonide 12

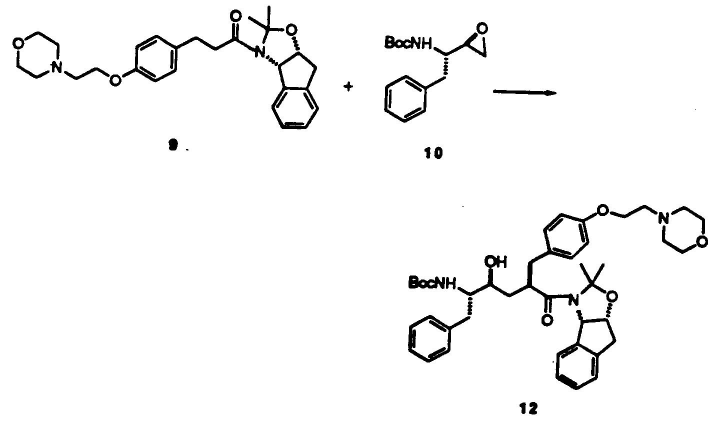

To a solution of the gummy amide **9** (902 mg, 2.00 mmol) in 10 mL sieve dried THF at -78°C was added nBuLi in hexanes (1.25 mL, 2.00 mmol). The bright yellow solution was aged at -78°C for 1h. In a separate flask, a solution of isopropylmagnesium chloride (1.00 mL, 2.00 mmol) was added to a -78°C solution of the epoxide **10** (527 mg, 2.00 mmol, 93:7 mixture (R:S) epoxides) in 8 mL sieve dried THF. After 15 min, the -78°C epoxide solution was transferred via canula to the -78°C amide enolate solution, then the mixture was warmed to -25°C and aged for 3 h. The mixture was then warmed to 25°C and aged for 13 h. The reaction was quenched with 25 mL water, and extracted with ethyl acetate (2 x 40 mL), washed with saturated aqueous sodium chloride (1 x 15 mL), dried (MgSO₄) and concentrated in vacuo. Chromatography of the crude mixture on silica gel (80g) eluting with 3% methanol in ethyl acetate gave 796 mg (60%) of the desired protected isostere acetonide **12** as a white foam.
¹³C NMR (75.5 MHz, CDCl₃, major rotamer) δ_{c} 172.9, 157.5, 156.4, 140.6, 140.4, 138.2, 131.8, 130.3, 129.3, 128.5, 128.0, 126.9, 126.5, 125.5, 124.0, 114.6, 96.6, 79.6, 79.0, 67.8, 66.9, 65.7, 65.5, 57.6, 57.2, 54.0, 44.0, 38.2, 38.0, 37.2, 36.1, 28.3, 26.4, 23.9.

### EXAMPLE 6

### Preparation of hydroxy-amide 13

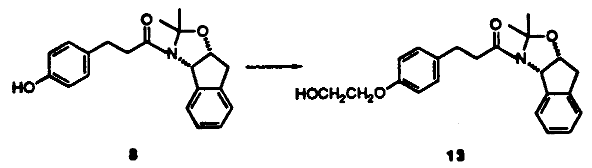

To a solution of the phenol **8** (1.86 g, 5.51 mmol) in 50 mL sieve dried acetonitrile at 25°C was added powdered K₂CO₃ (916 mg, 6.63 mmol) followed by ethyl bromoacetate (673 µL, 6.07 mmol). The resulting mixture was aged for 2 days at 25°C. The suspension was filtered and the filter cake was washed with acetonitrile and the organic phase was concentrated in vacuo, and the residue was azeotroped with THF (2 x 50 mL) and dried at high vacuum. The residue was dissolved in 55 mL sieve dried THF at 25°C and treated with 707 mg of LiBH₄ (32.5 mmol). The resulting mixture was aged at 25°C for 19 h and quenched with 20 mL of ethyl acetate at 25°C. The mixture was partitioned with 100 mL of ethyl acetate and 50 mL of water, the layers were separated and the aqueous layer was extracted with ethyl acetate (2 x 100 mL). The combined organic phase was washed with 50 mL water, 50 mL of saturated aqueous NaCl and dried over MgSO₄ Removal of the volatiles in vacuo gave 2.2 g crude product that was purified by chromatography on 65 g of silica gel. Elution with hexanes/ethyl acetate (1:2) gave 2.07 g (98%) of the hydroxy-amide **13** as a white foam.

### EXAMPLE 7

### Preparation of Isostere acetonides 14, 15and 16

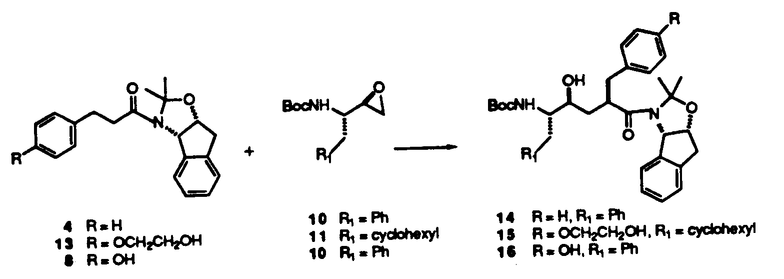

To a solution of epoxide **10** (159 mg, 0.604 mmol, 94:6 mixture of (R/S) epoxides) and amide **8** (194 mg, 0.604 mmol) in 4 mL sieve dried THF at -78°C was added a solution of nBuLi in hexanes (760 µL, 1.21 mmol). The-resulting yellow solution was aged at -78°C for 1 h, then warmed to -25°C and aged for 2h. The reaction was quenched by addition of water (5mL) at -20°C, and the mixture was extracted with ethyl acetate (2 x 40 mL). The combined ethyl acetate layer was washed with saturated sodium chloride, dried (MgSO₄) and concentrated in vacuo. The crude product (365 mg) was purified by chromatography on 18 g silica gel eluting with hexanes/ethyl acetate (2:1 to 1:1) to afford 306 mg (92% based on (R)-epoxide) of the product **14**. Trituration from hexanes/ethyl ether gave isostere acetonide **14** as a white solid.
¹³C NMR (100.6 MHz, CDCl₃, major rotamer) δ_{c} 172.9, 156.5, 140.7, 140.4, 139.5, 138.3, 129.4, 128.7, 128.6, 128.1, 127.2, 126.7, 126.6, 125.6, 124.1, 96.7, 79.7, 79.1, 68.0, 65.7, 57.4, 43.9, 38.4, 38.2, 38.1⁵, 36.2, 28.4, 26.5, 24.0.

To a solution of epoxide **10** (150 mg, 0.572 mmol 93:7 mixture of R/S epoxides) and amide **8** (194 mg, 0.575 mmol) in 2 mL of sieve dried THF at -78°C was added nBuli in hexanes (1.09 mL, 1.73 mmol) while keeping the temperature below -69°C. After the nBuLi addition, 4 mL of THF was added to the thick slurry to facilitate stirring. The yellow slurry was aged at -78°C for 1h, -50°C for 1h, and -20°C for 17 h. The thick mixture was quenched with 10mL of water, extracted with ethyl acetate (2x30 mL) and the organic phase was washed with saturated aqueous NaCl, dried (MgSO₄) and concentrated in vacuo. The crude product was purified by chromatography on 17 g of silica gel. Elution with hexanes/ethyl acetate (2:1) gave 207 mg (65% based on (R) epoxide) of the isostere acetonide **16** as an oil.
¹³C NMR (75.5 MHz, DMSO-d₆) δ_{c} 173.3, 156.6, 155.2, 140.3, 140.2, 138.1, 130.7, 130.4, 129.3, 128.5, 128.1, 127.0, 126.5, 125.5, 124.1, 115.6, 96.7, 79.9, 79.1, 68.2, 65.6, 57.2, 44.1, 38.1, 37.7, 37.4, 36.0, 28.2, 26.4, 23.9.

Use of the above procedure with epoxide **11** (512 mg, 1.90 mmol) and amide **13** (740 mg, 1.94 mmol) gave after purification by chromatography on 72 g of silica gel and elution with hexanes/ethyl acetate (1:1), 655 mg (53%) of the isostere acetonide **15** as a solid foam.
¹³C NMR (75.5 MHz, CDCl₃, major rotamer) δ_{c} 172.9, 157.6, 156.7, 140.8, 140.4, 132.4, 130.6, 128.1, 126.9, 125.6, 124.2, 114.7, 96.7, 79.6, 79.2, 70.9, 69.3, 65.7, 61.4, 53.3, 44.1, 39.2, 38.4, 37.9, 36.2, 34.4, 33.8, 32.9, 28.4, 26.6, 26.4, 26.2, 24.1.

### EXAMPLE 8

### Preparation of isosteres 17, 18, 19and 20

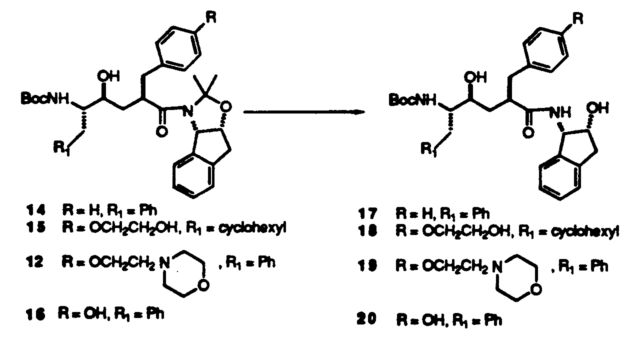

To a solution of the crude acetonide derivative **14** (2.00 g, 3.42 mmol) in 10 mL methanol at 0°C was added camphorsulfonic acid (200 mg, 0.86 mmol) followed by ethylene glycol (382 µL, 6.8 mmol). The mixture was warmed to 25°C and aged for 24 h (15 mL of methanol was added to the thick slurry after 2h to facilitate stirring). The slurry was cooled to 0°C, filtered thru a sintered glass funnel, and washed with 10 mL of cold methanol, and dried at high vacuum to afford 1.20 g (75% overall form (R)-epoxide) of the isostere **17** (Vacca, et al., J. Med. Chem. 1991, 34: 1228) as a white solid.
¹³C NMR (75.5 MHz, DMSO-d₆) δ_{c} 174.6, 155.3, 142.2, 140.6, 140.0, 139.7, 129.0, 128.9, 128.0, 127.9, 127.0, 126.1, 125.8, 125.7, 124.7, 124.1, 77.3, 72.1, 68.9, 56.6, 56.3, 43.8, 39.7, 38.9, 35.7, 35.4, 28.2.

Use of the above procedure with acetonide derivative **15** (2.13 g, 3.27 mmol) gave 1.21 g (61%) of the deblocked isostere **18** as a powdery solid.
¹³C NMR (75.5 MHz, DMSO-d₆) δ_{c} 174.8, 156.8, 155.5, 142.3, 140.6, 131.8, 129.7, 127.0, 126.0, 124.7, 124.0, 114.0, 77.2, 72.1, 69.7, 69.3, 59.5, 56.6, 51.8, 44.0, 39.8, 38.0, 37.7, 35.7, 33.8, 33.4, 32.1, 28.2, 26.1, 25.9, 25.8.

Use of the above procedure with acetonide derivative **12** (31 mg, 0.043 mmol) gave 17.5 mg (60%) of the deblocked isostere **19** as a white solid.
¹³C NMR (75.5 MHz, DMSO-d₆) δ_{c} 174.8, 156.7, 155.4, 142.2, 140.6, 139.7, 131.9, 129.8, 129.0, 128.0, 127.1, 126.2, 125.7, 124.7, 124.2, 114.1, 77.4, 72.2, 68.9, 66.2, 65.2, 57.1, 56.7, 56.3, 53.6, 44.1, 39.8, 38.1, 35.8, 35.3, 28.2.

Use of the above procedure with acetonide derivative **16** gave the deblocked isostere **20**.

## Claims

1. A process for making a compound of structural formula : 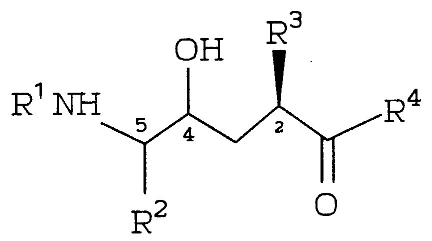 which comprises reacting an epoxide of formula: 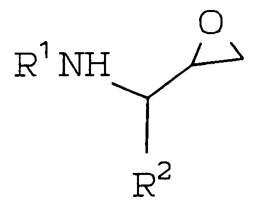 with about one equivalent of an amide of structural formula: 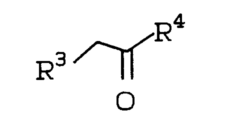 by treatment with a strong base at low temperatures wherein:
R¹ is:
a) t-Boc, Aoc, Adc, Mcb, Mch, Cbz, Alloc, or Fmoc,
b) benzyl,
c) lower alkyl,
d) CH₃O(CH₂CH₂O)ₙCO-, wherein n is from 1 to 10,
e) tetrahydrofuranyloxy-CO-, or
f) R-CO-, wherein R is an amino acid;
R² is:
a) -H
b) benzyl,
c) cyclohexyl-CH₂-,
d) isobutyl,
e) benzyl-O-benzyl-,
f) cinnamyl-, or
f) parahydroxybenzyl;
R³ is:
a) hydrogen,
b) lower alkyl,
c) phenyl
d) benzyl,
e) -CH₂-phenyl-O-R⁵, or
f) -CH₂-CH=CH-phenyl-O-R⁵;
R⁴ is:
a) 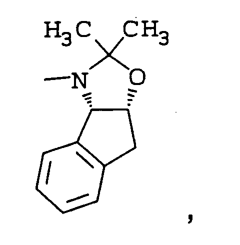
b) 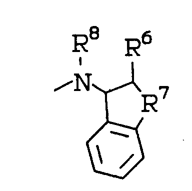 wherein:
R⁶ is -H, or -OH;
R⁷ is -CH₂-O-, or 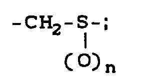 n = 0, 1, or 2;
c) 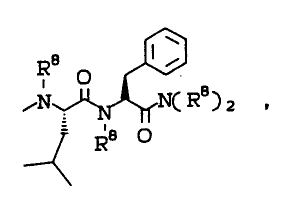
d) 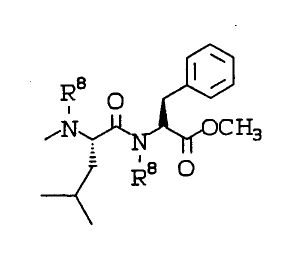
e) 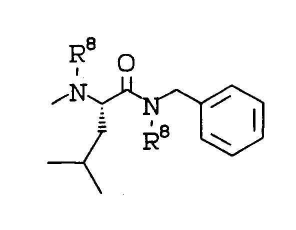
f) 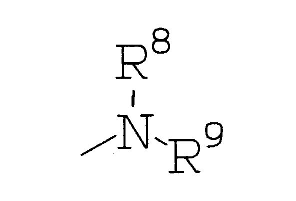
g) 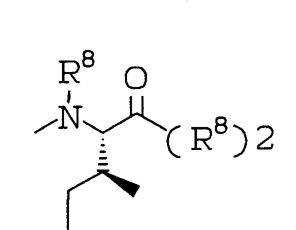 or
h) 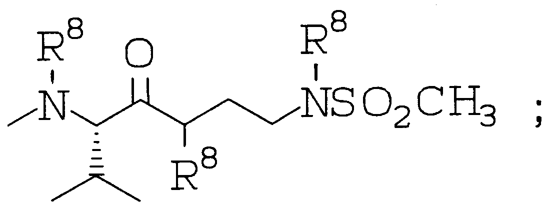
R⁵ is:
a) -H
b) benzyl,
c) -CH₂CH₂-OH, or
d) 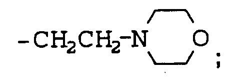
R⁸ is a removable protecting group selected from among:
a) t-Boc, Aoc, Adc, Mcb, Mch, Cbz, Alloc, or Fmoc, or
b) benzyl; and
R⁹ is:
a) lower alkyl, or
b) benzyl.

2. The process of Claim 1 wherein :
R¹ is t-Boc, Aoc, Adc, Mcb, Mch, Cbz, Alloc, or Fmoc;
R² is -H, benzyl, cyclohexyl-CH₂-, or parahydroxybenzyl;
R³ is phenyl, benzyl, or -CH₂-phenyl-O-R⁵; and
the strong base is chosen from the group consisting of n-butyllithium, s-butyllithium, t-butyllithium, potassium tert-butoxide, lithium diisopropylamide, lithium isopropylcyclohexylamide, lithium pyrrolidide, lithium tetramethylpiperidide, phenyllithium, isopropylmagnesium chloride, and isobutylmagnesium chloride.

3. The process of Claim 1 for making a compound of strucural formula: 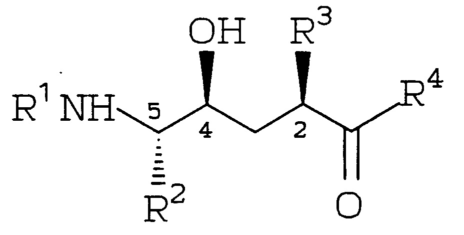 2R, 4S, 5S diastereomer
which comprises reacting an epoxide of formula: 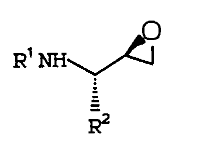 with about one equivalent of an amide of formula:

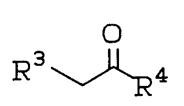

4. The process of Claim 3 wherein:
R¹ is t-Boc, Aoc, Adc, Mcb, Mch, Cbz, Alloc, or Fmoc;
R² is -H, benzyl, cyclohexyl-CH₂-, or parahydroxybenzyl;
R³ is phenyl, benzyl, or -CH₂-phenyl-O-R⁵; and
R⁴ is 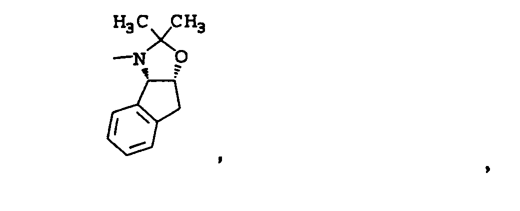 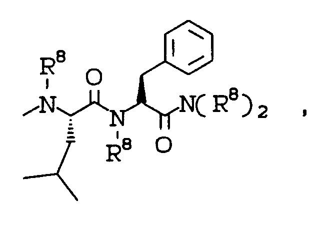 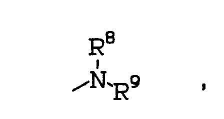 or

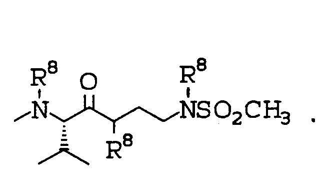

5. The process of Claim 4 wherein the strong base is chosen from the group consisting of n-butyllithium, s-butyllithium, lithium diisopropylamide and isopropylmagnesium chloride.

6. The process of Claim 5 wherein the low temperature is in the range of about -82°C to -65°C to effect metalation of the amide and the epoxide, and the temperature is in the range of about -50°C to -10°C to effect the reaction of the metalated derivatives of the amide and the epoxide.

7. The process of Claim 6 wherein:
R¹ is t-Boc;
R² is benzyl or cyclohexyl-CH₂-;
R³ is benzyl or -CH₂-phenyl-O-R⁵;
R⁴ is: 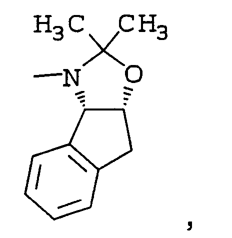 and
R⁵ is -H, -CH₂CH₂-OH, or

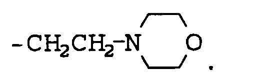

8. The process of Claim 7 wherein the strong bases are chosen from the group consisting of n-butyllithium and isopropylmagnesium chloride.

9. The process of Claim 7 wherein R⁴ is deprotected to the free amino alcohol.

10. A compound of structural formula: 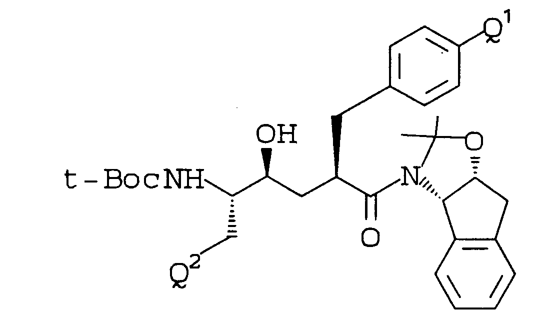 wherein Q¹ is
a) -H,
b) -OH,
c) 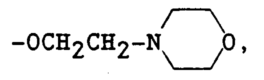 or
d) -OCH₂CH₂OH; and
Q² is
a) phenyl or
b) cyclohexyl.
